# EUROPEAN PATENT APPLICATION

(11) **EP 1 505 389 A1**
(43) Date of publication of application: **09.02.2005**
(21) Application number: 03254873.7
(22) Date of filing: 05.08.2003
(51) Int. Cl.: G01N 33/00, G01N 33/497

(54) **Personal and environmental fluid sampling apparatus**

(71) Applicant: INDUSTRIAL TECHNOLOGY RESEARCH INSTITUTE, Hsin-Chu Hsien 311 (TW)
(72) Inventor: Kao, Hsi Feng, Junghe City Taipei 235 (TW); Chung, Cheng Shiu, Hsinchu (TW); Lin, Yuh-Jiuan, Taishan Shiang Taipei (TW)
(74) Representative: Stonehouse, Sidney William

(57) **Abstract**

A portable sampling apparatus (1) comprising: a housing; a sample inlet (2) mounted in the housing; a sensor module (5) mounted in the housing, and a sampling device comprising a reservoir (3). The sampling device is used for the transporting of the sample and controls the flow rate of the sample.

To be accompanied, when published, by Figures 1A and 3 of the drawings.

## Description

### BACKGROUND OF THE INVENTION

### 1. Field of the Invention

The present invention relates to a sampling device and, more particularly, to a sampling device with built-in reservoirs and pumps.

### 2. Description of Related Art

Nowadays, samples for some sampling devices used for diagnosis is taken through patients' blowing or breath. The inlet tubes of these sampling devices are disposed and replaced after use in order to prevent the prevailing of contagious diseases and to maintain the devices to be clean. However, during the operation for replacing the inlet tubes, the operators still have chance to contact the patients' saliva and then indirectly pass the saliva to the new inlet tubes, which may cause the prevailing of the contagious diseases.

In addition, since most inlet tubes of these sampling devices are designed as an extended tube protruding from the outer case of the sampling devices, the inlet tubes of these sampling devices are easy to be contaminated as the sampling devices dropped on the ground or in some polluted environment. In some cases, the contaminated inlet tubes also cause serious errors or inaccuracy for detection.

On the other hand, when a sampling device is used in an environmental detection, especially for the special cases such as the gas leakage in the piping in a factory, the inlet tube may be stained by some hazard or toxicant. If the operators replace the inlet tubes by hands, the hazards or toxicants may cause serious injuries.

In some special cases, it is required to detect and analyze the sample in a long-term, constant flow-rate mode so as to provide more accurate result. A good example is the breathalyser. The result made by a breathalyser is based on the amount of alcohol vapors contained in one's breath. However, since the volume of each breath is different even for the same single person, thus the test results based on different breaths are not the same and depend on how deep the breaths are.

Among the commercial sampling devices one can find in the market, there is no such mechanism that can hold and receive the sample inlet tube, nor is there the mechanism to make the inlet tube detach from the apparatus without the contact of the operator.

Recently, a sampling method requiring operators to analyze the test result is disclosed in US patent 4,947,861. The sampling method in US patent 4,947,861 cannot be applied with constant sample flow-rate. Another sensing apparatus comprising a housing, a sensor module and a sample chamber to hold the sample is disclosed in US patent 6,234,006. However, the apparatus in US patent 6,234,006 can't be used for an inconstant flow-rate detection because there is no room for holding the sample and then transmitting the sample to the sensor module in a constant flow-rate. But for most illness detection or for the operation of hyper-sensitivity sensing apparatus, it is necessary to provide a space for sample storage to transmitting the sample to the sensor module constantly.

Therefore, it is desirable to provide an improved speech recognition method to mitigate and/or obviate the aforementioned problems.

### SUMMARY OF THE INVENTION

An object of the present invention is to provide a sampling apparatus capable to hide or hold the sample inlet tube to prevent contamination of the inlet tube and the apparatus, and to reduce the size of the whole apparatus.

Another object of the present invent is to provide a sampling apparatus which comprises a sampling device having a reservoir so as to provide a constant flow-rate sample for detection.

Another object of the present invention is to provide an analyzer with reduced size for preventing possible contamination from outside environment.

To achieve the object, the present invention also provides a portable sampling apparatus used for detecting and analyzing a sample, includes: a housing; and a sample inlet mounted on the housing, wherein said sample inlet can rotate, flip-flop, slide, fold, or extend to be received into said housing.

To achieve the other object, the sampling apparatus of the present invention used to detect a sample, includes: a housing; a sample inlet mounted in said housing; a sensor module mounted in said housing; and a sampling device comprising a reservoir, said sampling device is used for the transporting of the sample and controls the flow rate of the sample.

The analyzer of the present invention includes: a housing; and a sample inlet mounted on the housing, wherein said sample inlet is able to extend out from said housing through rotation, flip-flopping, sliding, folding or springing.

Other objects, advantages, and novel features of the invention will become more apparent from the following detailed description when taken in conjunction with the accompanying drawings.

### BRIEF DESCRIPTION OF THE DRAWINGS

FIG. 1A shows a 3-D view of the first embodiment where the inlet tube is folded in the concave.
FIG 1B shows a 3-D view of the first embodiment where the inlet tube is unfolded.
FIG 2A shows a 3-D view of the second embodiment where the inlet tube is received in a receiving hole.
FIG. 2B shows a 3-D view of the second embodiment where the inlet tube flips out of the receiving hole.
FIG. 3 shows a diagram of the third embodiment of the micro-valve connected to the first pipe.
FIG. 4 shows a diagram of the third embodiment of the micro-valve connected to the intersection of the first and second pipes.
FIG. 5 shows a diagram of the fourth embodiment of a first micro-valve connected to the first pipe and a second micro-valve connected to the third pipe.
FIG. 6 shows a diagram of the fifth embodiment of the reservoir being connected to the pump by the third pipe through a second opening.
FIG. 7 shows a diagram of the sixth embodiment of a first valve connected to the first pipe and a second valve connected to the third pipe, with the reservoir comprising a second opening connecting to the pump through the third pipe.

### DETAILED DESCRIPTION OF THE PREFERRED EMBODIMENT

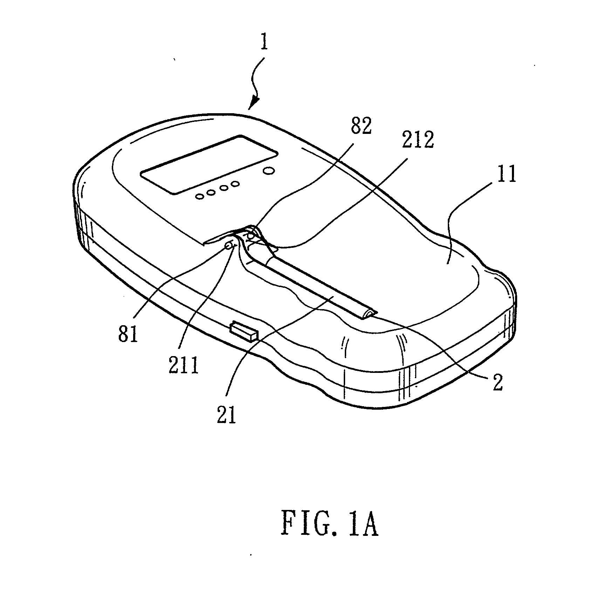

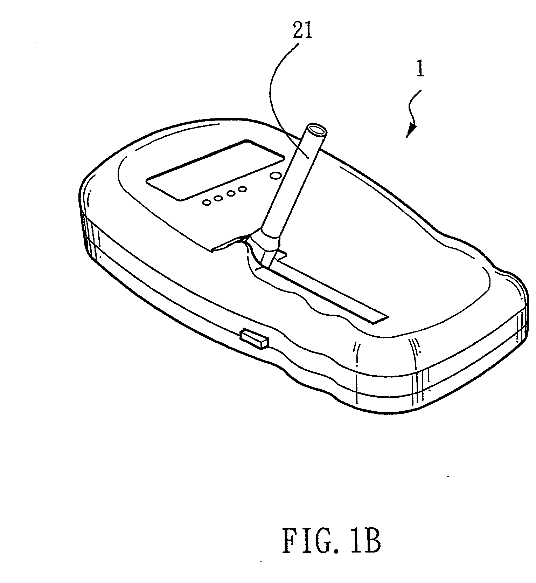

Please refer to the FIG. 1A of the present invention. A schematic view of the first embodiment of the sampling apparatus is shown in FIG. 1A. The inlet tube 21 of the sampling apparatus can be received and folded in the concave 8 of the sampling apparatus. The inlet tube 21 can be received in a concave 8 or be extended from the concave 8 through folding means. In the present embodiment, the folding mean is the combination of holes 81, 82 and protrusion pins 211,212. As shown in FIG. 1A, two holes 81, 82 opposing each other can be found at one end of the concave 8 of the housing. On the other side, two protrusion pins 211,212 corresponding to the two holes 81 are arranged to connect the holes 81, 82 on the housing. The inlet tube 21 is mounted on the housing. The two protrusion pins 211,212 inserting into the two holes function as a hinge, and thereby the inlet tube 21 can rotate by the hinge, as shown in FIG. 1B.

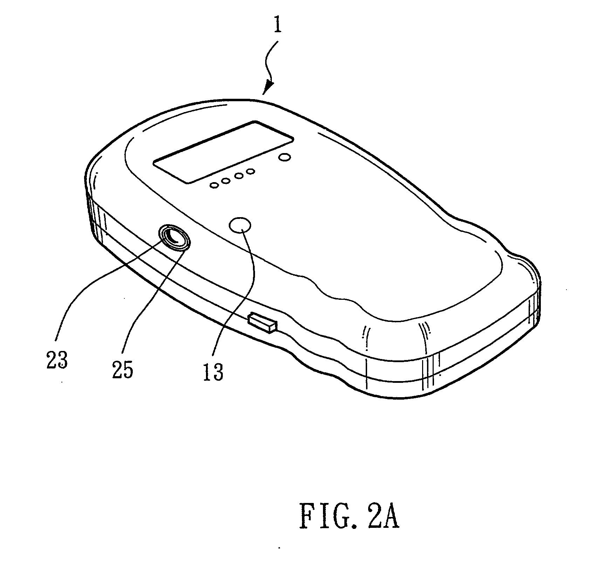

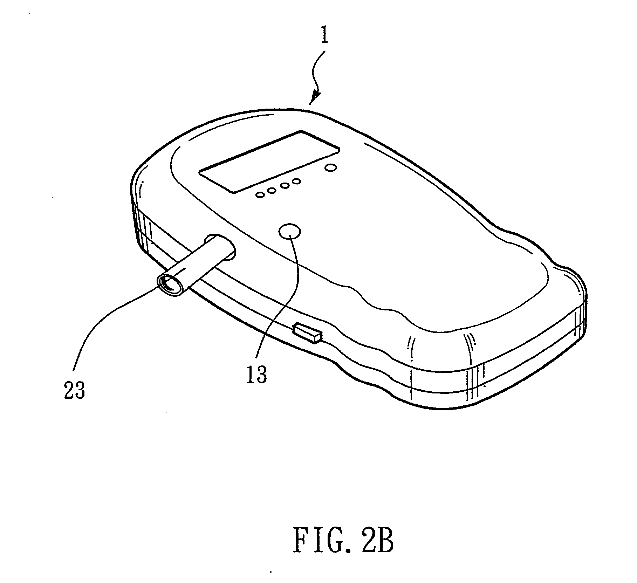

Please refer to FIG. 2A of the present invention, which shows a schematic view of the second embodiment. The inlet tube 23 of the sampling apparatus can be received in the receiving hole 25 by a holding means (not shown in the figure) when it's not applied. To make the inlet tube 23 get ready for use, the releasing button 13 are pushed or triggered to release the holding means, and then the inlet tube flipps out from the receiving hole 25, as shown in FIG 2B. Other ways for receiving and holding the inlet tube in the housing of the sampling apparatus can be conceived, but not limited Preferably, these ways include sliding in and out, folding up, stretching out, or compressing in the inlet tube.

In the sampling apparatus of the present invention, the passing of the fluid sample is controlled by valves. Preferably, the passing of the fluid sample is controlled through electrical micro-valves. The sample is stored in a reservoir as the micro-valves between the inlet tube and the reservoir are opened, the micro-valves between the reservoir and the pump are closed. The micro-valves between the reservoir and the pump are then opened and the micro-valves between the inlet tube and the reservoir are closed as a micro-pump creates pressure difference to transmit the sample from the reservoir to the sensor module through the pipes. All the actuation of the micro-valves and the micro-pumps are controlled by a microprocessor.

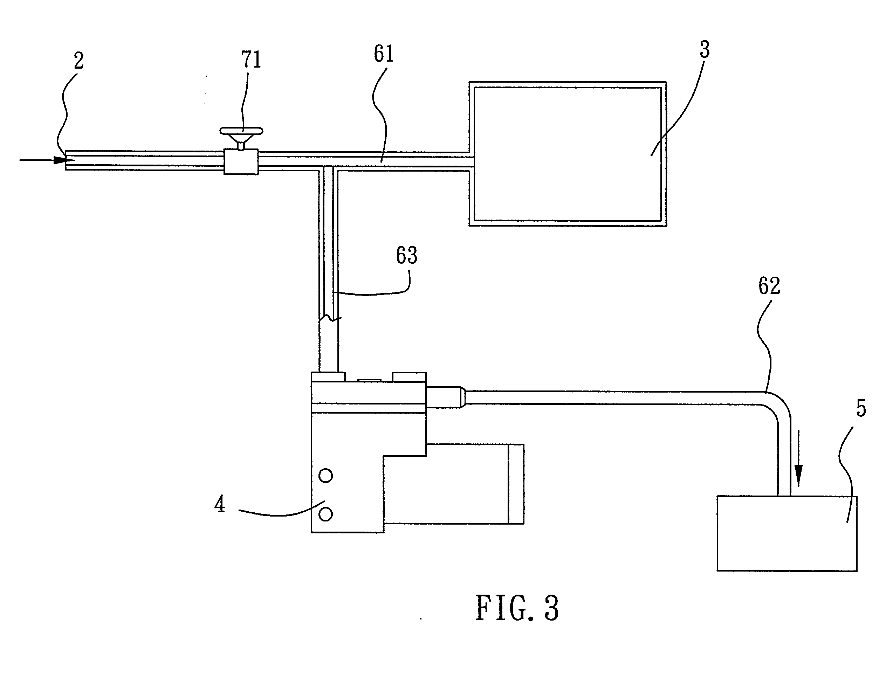

The following five embodiments from the second to the sixth are based on the similar arrangement. They are different to each other only in the arrangement in piping and valve setting can be found. The third, fourth and fifth embodiments has one thing in common that the reservoir 3 comprises only one opening 31. Please refer to FIG. 3. This is a diagram of the third embodiment showing the components of the sampling apparatus comprising a sample inlet 2, a reservoir 3, a micro-pump 4, a sensor module 5, a first pipe 61, a second pipe 62, a third pipe 63 and a micro-valve 71, wherein the sample inlet 2 is connected to the reservoir 3 by the first pipe 61 through an opening 31 on the reservoir so that the sample coming from the sample inlet 2 can be stored in the reservoir 3. The micro-pump 4 is connected to the sensor module by the second pipe 62 and connected to the first pipe 61 by the third pipe 63. The micro-valve 71 is connected to the first pipe 61 at a position between the sample inlet 2 and the intersection of the first pipe 61 and the third pipe 63 so as to control the amount of the sample fluid. The microprocessor (not shown in the figure) will turn off the micro-valve 71 and turn on the micro-pump 4 to pump the sample fluid stored in the reservoir 3 to the sensor-module 5 at a constant flow rate. The microprocessor and a D/A converter (digital/analogous converter) works together to convert the digital signals to analogous signals, and then linearly amplify the analogous signals by an operational amplifier to provide the amplified voltage to drive the micro-pump 4. Therefore, the micro-pump 4 can be driven by a digital microprocessor. In this way, the sample can be pumped to the sensor-module by the operation of the micro-pump 4. By actuating micro-valve 71 and the micro-pump 4 at the same time, the sample can be transmitted from the sample inlet directly to the sensor-module 5 without storing in the reservoir 3. One can also "clean up" the sampling apparatus by simply replace the sample with a clean fluid (e.g. clean air).

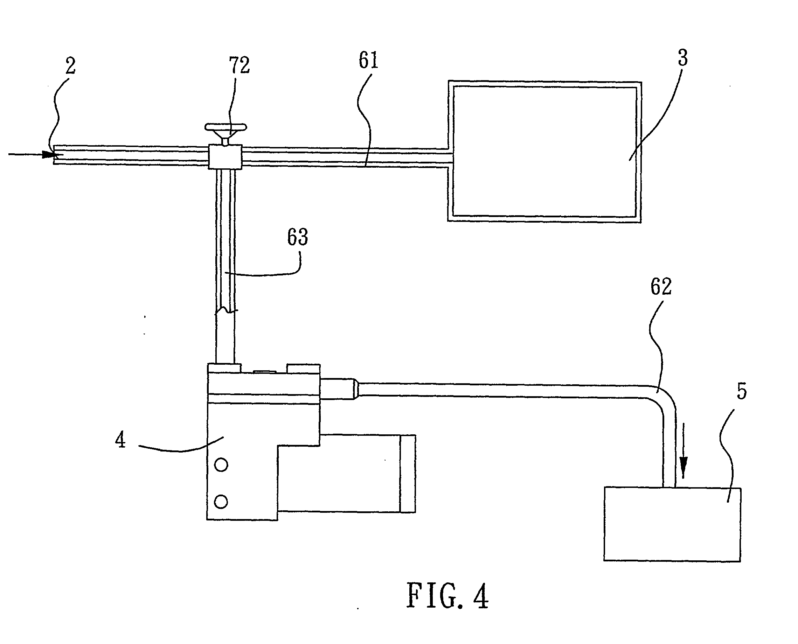

Please refer to FIG. 4. This is the diagram of the fourth embodiment showing the modification of the third embodiment. Unlike FIG. 3, the micro-valve 72 is a 3-way valve having a first exit port 721 directing to the reservoir and a second exit port 722 directing to the micro-pump 4. The micro-valve is connected to the first pipe 61 right at the intersection of the first pipe 61 and the third pipe 63. Therefore when the operator is doing sampling, the microprocessor turns on the first exit port 721 and turns off the second exit port 722. When doing analyzing, the microprocessor turns off the first exit port and turns on the second exit port 722 so as to pump the sample from the reservoir 3 to the sensor-module 5 at a constant flow rate.

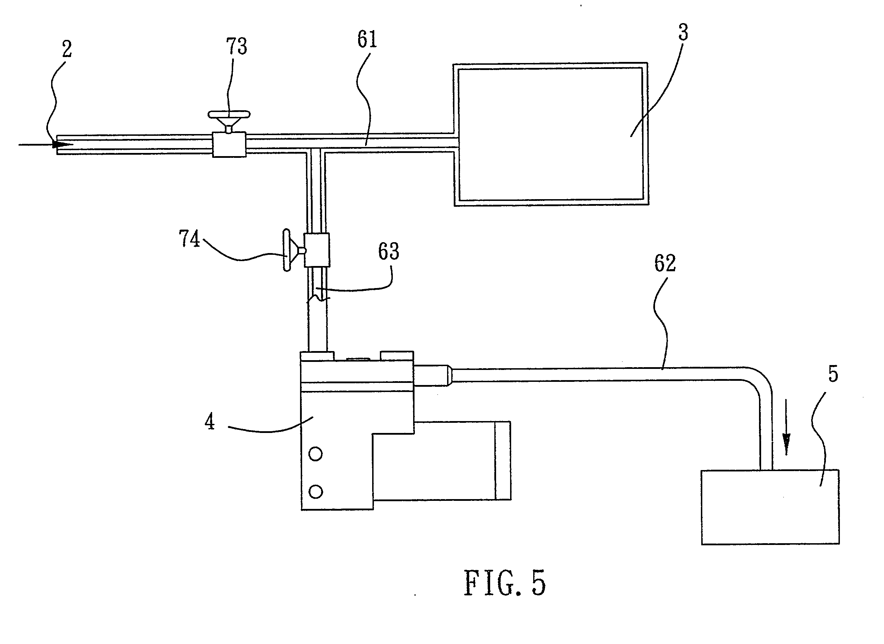

Please refer to FIG 5. This is the diagram of the fifth embodiment showing another modification of the third embodiment. The first micro-valve 73 in this figure remains at its position as in FIG 3. However, another connection between a second micro-valve 74 and the third pipe 63 is made. Therefore when doing sampling, the microprocessor turns on the first micro-valve 73 and turns off the second micro-valve 74 so that the sample can be conducted to the reservoir 3 rather than to the pump 4. When doing analyzing, the microprocessor turns off the first micro-valve 73 and turn on the second micro-valve 74 so as to pump the sample from the reservoir 3 to the sensor-module 5 at a constant flow rate.

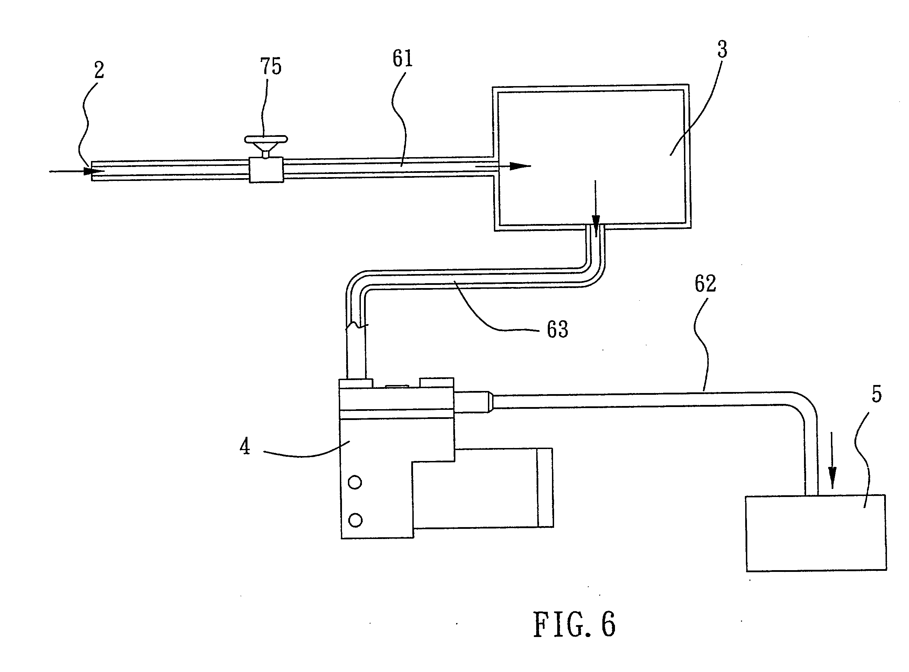

The sixth and seventh embodiments are another type of arrangement of the piping and valves wherein the reservoir 3 comprises a first opening 32 and a second opening 33 so that the sample comes into the reservoir through the first opening 32 and being pumped out of the reservoir through the second opening 33. Please refer to FIG. 6. This is the diagram of the sixth embodiment. A first micro-valve is connected to the first pipe 61, and the first pipe 61 is connected to the reservoir 3 through a first opening 32. The reservoir 3 is connected to the micro-pump 4 through a second opening 33.

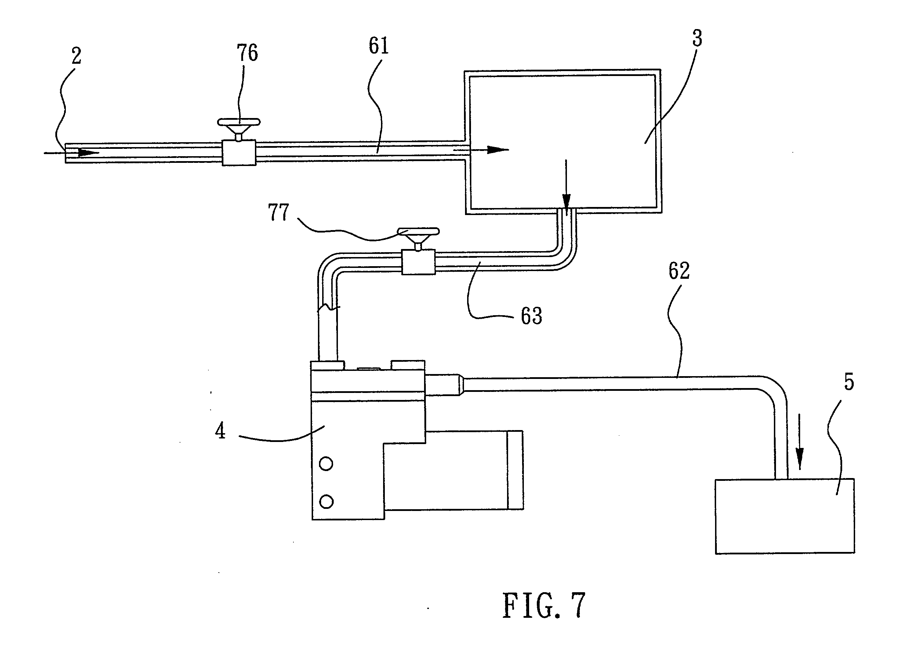

Please refer to FIG. 7. This is the diagram of the seventh embodiment which shows a modification of the sixth embodiment. The difference of the sixth and the seventh embodiment lies in that there is a second micro-valve 77 connecting to the third pipe 63 in the seventh embodiment. Therefore when doing sampling, the microprocessor turns on the first micro-valve 76 and turns off the second micro-valve 77 so that the sample stops and being stored in the reservoir 3. When doing analyzing, the microprocessor turns off the first micro-valve 76 and turns on the second micro-valve 77 so as to pump the sample from the reservoir 3 to the sensor-module at a constant flow rate.

From the illustration of the above embodiments, the advantages of foldable sample inlet tubes can be amplified to reduce or prevent any possible contamination. Beside, the embedded sample inlet tubes also reduce the size of the sampling apparatus and make the sampling apparatus much more convenient for carrying. In addition, the arrangement of the reservoir-pump design can provide a sample fluid in a relatively constant flow rate. Therefore, constant-rate or non-constant-rate sampling can be achieved smoothly since the buffering of the built-in reservoir can provide a fluid in a relatively constant flow rate to the sensor module. The sampling apparatus of the present invention provides a more stable and accurate result, as well as a faster analyzing time.

Although the present invention has been explained in relation to its preferred embodiment, it is to be understood that many other possible modifications and variations can be made without departing from the spirit and scope of the invention as hereinafter claimed.

## Claims

1. A sampling apparatus (1) comprising:
a housing and a sensor module (5), **characterised in that** a sample inlet (2, 21, 23) is mounted in said housing;
the sensor module (5) is mounted in said housing; and
a sampling device is provided for transporting a sample, and controlling the flow rate of the sample.

2. The sampling apparatus (1) as claimed in claim 1, **characterised in that** said sample is a fluid sample, said sampling device has at least a reservoir (3) mounted in said housing for storing said fluid sample, and said reservoir is connected to said sample inlet (2).

3. The sampling apparatus (1) as claimed in claim 2, **characterised in that** at least one pump (4) is mounted between said reservoir (3) and said sensor module (5) for transmitting the fluid sample from said reservoir to said sensor module.

4. The sampling apparatus (1) as claimed in claim 3, **characterised in** further comprising a first pipe (61) located between said sample inlet (2) and said reservoir (3), wherein said first pipe (61) connects said reservoir (3) through a first opening (31) thereon.

5. The sampling apparatus (1) as claimed in claim 5, **characterised in** further comprising a second pipe (62) located between said pump (4) and said sensor module (5).

6. The sampling apparatus (1) as claimed in claim 5, **characterised in** further comprising a third pipe (63) located between said pump (4) and said first pipe (61), said pump (4) being used for transmitting said fluid sample from said reservoir (3) to said sensor module (5).

7. The sampling apparatus (1) as claimed in claim 6, **characterised in** further comprising a first valve (71, 72) mounted at a position between said sample inlet (2) and the intersection of said first pipe (61) and third pipe (63).

8. The sampling apparatus (1) as claimed in claim 7, **characterised in that** said first valve (72) is a 3-way valve mounted on the intersection of said first pipe (61) and said third pipe (63).

9. The sampling apparatus (1) as claimed in claim 7, **characterised in** further comprising a second valve (74) mounted on said third pipe (63).

10. The sampling apparatus (1) as claimed in claim 5, **characterised in that** said reservoir (3) is connected to said pump (4) by a third pipe (63) through a second opening (32) of said reservoir.

11. The sampling apparatus (1) as claimed in claim 10, **characterised in that** a first valve (76) is mounted on said first pipe (61).

12. The sampling apparatus (1) as claimed in claim 11, **characterised in that** a second valve (77) is mounted on said third pipe (63).

13. The sampling apparatus (1) as claimed in claim 1, **characterised in that** said sampling apparatus is used as a sensor.

14. The sampling apparatus (1) as claimed in claim 1, **characterised in that** said sampling apparatus is used as an analyser.

15. A sampling apparatus (1) comprising a housing and **characterised in that** a sample inlet (23) mounted on said housing, is able to extend out from said housing through rotation, flip-flopping, sliding, folding or springing.
